(19) Europäisches Patentamt
European Patent Office
Office européen des brevets

(11) **EP 1 226 841 B1**

(12) **EUROPÄISCHE PATENTSCHRIFT**

(45) Veröffentlichungstag und Bekanntmachung des Hinweises auf die Patenterteilung:
**25.01.2006 Patentblatt 2006/04**

(51) Int Cl.:
*A61M 16/00* (2006.01)

(21) Anmeldenummer: **02001880.0**

(22) Anmeldetag: **28.01.2002**

(54) **Verfahren und Vorrichtung zur Schlafüberwachung**

Method and device for sleep monitoring

Méthode et dispositif de surveillance du sommeil

(84) Benannte Vertragsstaaten:
**AT BE CH CY DE DK ES FI FR GB GR IE IT LI LU MC NL PT SE TR**

(30) Priorität: **30.01.2001 DE 10103973**

(43) Veröffentlichungstag der Anmeldung:
**31.07.2002 Patentblatt 2002/31**

(73) Patentinhaber:
• **Wirtz, Hubert, Dr.**
**04416 Markkleeberg (DE)**
• **Kowallik, Lydia, Dr.**
**97076 Würzburg-Lengfeld (DE)**

(72) Erfinder:
• **Kowallik, Peter L., Dr.**
**97076 Lengfeld (DE)**

• **Wirtz, Hubert, Dr.**
**04416 Markkleeberg (DE)**

(74) Vertreter: **Heusler, Wolfgang**
**v. Bezold & Sozien**
**Patentanwälte**
**Akademiestrasse 7**
**80799 München (DE)**

(56) Entgegenhaltungen:
WO-A-93/09834          US-A- 4 228 806
US-A- 5 101 831          US-A- 5 353 788

• LOUBE D L; GAY P C, STROHL K P; PACK A I; WHITE D P; COLLOP N A: "Indications for positive airway pressure treatment of adult obstructive sleep apnea patients: A consensus statement" CHEST, Bd. 115, Nr. 3, März 1999 (1999-03), Seiten 863-866, XP002258814 US

**Beschreibung**

Die Erfindung betrifft eine Vorrichtung zur Beatmung eines Patienten und/oder zur Signalisierung von Schlafzuständen.

Schlafbezogene Atmungsstörungen sind eine weit verbreitete Krankheit, die sich in verschiedenen Formen manifestieren kann. Häufig kommt es zur obstruktiven Schlafapnoe (OSA), die mit Tagesmüdigkeit und Kreislauferkrankungen einhergeht (siehe z. B. P. J. Strollo Jr. et al. in "N. Engl. J. Med.", Band 334, 1996, Seite 99). Bei der erkrankten, schlafenden Person kann es insbesondere infolge eines erhöhten Atemwegswiderstands (UAR) wiederholt zu einem Kollaps der oberen Atemwege kommen. Auch wenn es nicht zu einer vollständigen Behinderung der Atemwege kommt, können sich bei einem erhöhten UAR klinische Symptome zeigen.

Die gegenwärtige Diagnostik für die obstruktive Schlafapnoe umfaßt ein Screening, das die Apnoe recht gut erkennt, die sogenannte Hypopnoe (verminderter Atemfluß) jedoch nicht mit einer vergleichbaren Zuverlässigkeit. Es werden Messungen der Sauerstoffsättigung und des an Mund und Nase gemessenen Atemflusses durchgeführt. Diese Diagnostik wird in der Regel angewendet, wenn ein Patient über Tagesmüdigkeit klagt. Tagesmüdigkeit entsteht aber auch, wenn viele Mikro-Schlafunterbrechungen (Arousals) auftreten, die beim UAR-Syndrom (UARS) entstehen, weil die oberen Atemwege eng sind, ohne vollkommen verschlossen zu sein. Es muß vom Organismus ein relativ großer Druck aufgewendet werden, um den Atemfluß aufrechtzuerhalten. Große Druckschwankungen im Thorax sind vermutlich die Ursache für die Mikro-Aufwachreaktionen und auch für ungünstige Folgen für das Herz-Kreislaufsystem. Mit der herkömmlichen Diagnostik kann dieses Problem nicht erfasst werden, da es aufgrund des großen Druckes nicht zu einer wesentlichen Abschwächung des Atemstromes kommt. Eine Messung des Druckes wäre lediglich mit einem intraösophagialen Ballon möglich, der eine invasive und unangenehme Diagnostik für den Patienten bedeuten würde.

Zur Detektion des UARS ist es bisher üblich, den Ösophagusdruck zu messen und gleichzeitig Aufwachreaktionen zu detektieren (siehe z. B. C. Guilleminault et al. in "Chest" Band 104, 1993, Seite 781). Diese Verfahren sind jedoch wegen des hohen technischen Aufwandes und der erforderlichen Messzeit nachteilig und mit für den Patienten unangenehmen Eingriffen verbunden.

Es ist ferner bekannt, dass ein erhöhter UAR mit Veränderungen messbarer Zustandsgrößen der Atmung, wie z. B. des Druckverlaufs (siehe J.-J. Hosselet et al. in "Am. J. Respir. Crit. Care. Med.", Band 157, 1998, Seite 1461) oder den Längen des Atemzugs (siehe z. B. T. Brack et al. in "Am. J. Respir. Crit. Care. Med.", Band 157, 1998, Seite 1756), verbunden ist. Diese Erkenntnis hat jedoch bislang keine Anwendungen bei der Schlafüberwachung ergeben.

Patienten mit schlafbezogenen Atemstörungen werden bisher insbesondere mit einer sogenannten CPAP-Therapie behandelt (CPAP: Continuous Positive Airway Pressure). Bei der CPAP-Behandlung ist vorgesehen, dass der schlafende Patient laufend über eine Nasalmaske mit einem Beatmungs-Überdruck beaufschlagt wird. Der Überdruck wird mit einem Beatmungsgerät (CPAP-Gerät) erzeugt.

Die CPAP-Therapie besitzt den Nachteil, dass der Patient während der gesamten Dauer des Schlafes, also insbesondere für die ganze Nacht, einem Überdruck ausgesetzt ist. Dies bedeutet eine Belastung des Patienten. Um diese möglichst gering zu halten, wird ein herkömmliches CPAP-Gerät mit einem möglichst geringen Druck betrieben. Dies bedeutet allerdings ein Risiko. In Einzelfällen kann der Betriebsdruck des CPAP-Gerätes zu gering sein.

Die US-A-5 101 831 beschreibt ein Wecksystem, mit dem durch kontinuierliche Ermittlung der Schlafzustände eine für guten Schlaf angenehme, nicht in Tiefschlafstadien fallende Aufweckzeit bestimmt werden soll. Die Unterscheidung der Schlafstadien erfolgt durch Messung der Pulsfrequenz oder der Atemfrequenz und durch Berechnungen aufgrund von Änderungen dieses biologischen Signals und dessen Vergleich mit einem vorbestimmten Schwellwert.

Die US-A-5 353 788 beschreibt ein medizinisches System zur automatischen Steuerung des zur Behandlung von OSA-Problemen erforderlichen Beatmungsluftdrucks. Zu diesem Zweck werden mehrere unterschiedliche Parameter des Patienten gemessen und ausgewertet.

Die Aufgabe der Erfindung ist es, eine Vorrichtung zur Erfassung von Atmungsparametern eines schlafenden Probanden bereitzustellen, die sich insbesondere durch die Messung eines möglichst einfachen Signals mit einer möglichst geringen Belastung des Patienten auszeichnet und bei der Steuerung von Beatmungsgeräten oder der Signalisierung von Atmungsstörungen verwendbar ist.

Diese Aufgabe wird durch eine Vorrichtung mit den Merkmalen des unabhängigen Patentanspruchs gelöst. Vorteilhafte Ausführungsformen und Anwendungen der Erfindung ergeben sich aus den abhängigen Ansprüchen.

Die Grundidee der Erfindung ist die Erfassung von Atmungsparametern, die für den Atemzustand eines schlafenden Probanden charakteristisch sind, wobei der zeitliche Verlauf mindestens einer sich wiederholt mit der Atmung verändernden Zustandsgröße des Herz-KreislaufSystem des Probanden gemessen und einer Rhythmusanalyse unterzogen wird, um als Atmungsparameter statistische Werte der Verteilung von Atmungsintervallen in nicht-obstruierten Atmungsphasen oder davon abgeleitete Größen (Größen, die aus den statistischen Werten der Verteilung von Atmungsintervalle berechnet werden) zu ermitteln. Als Atmungsparameter werden z. B. die Variabilität oder der sog. Kurtosis-Wert der betrachteten Atmungsintervalle ermittelt. Als Zustandsgröße des Herz-Kreislaüf-Systems kann jede physikalische oder chemische Eigenschaft des Probanden gemessen werden, die sich zeitlich parallel mit dem Rhythmus des wiederholten Ein- und Ausatmens der Probanden verändert. Es erfolgt beispielsweise eine Messung des Gasflusses beim Atmen oder des Herzrhythmus. Die Rhythmusanalyse einer einzelnen mit der Atmung korrelierenden Zustandsgröße und die statistische Bewertung der zeitlichen Variabilität der Zustandsgröße in Zeitbereichen, in dem ein physiologisch normales Atmungsverhalten vorliegt, besitzt den Vorteil, dass ein einfaches, einkanaliges Signal ausgewertet wird, mit dem mit hoher Zuverlässigkeit charakteristische Schlafzustände des Probanden erfasst werden können.

Der gemäß der Erfindung erfasste mindestens eine Atmungsparameter kann zur Steuerung eines Beatmungsgerätes, insbesondere des Druckerzeugers eines Beatmungsgerätes, verwendet werden. Die Erfinder haben überraschenderweise festgestellt, dass die erfindungsgemäß ermittelten Atmungsparameter geeignet sind, den Betriebszustand von Beatmungsgeräten insbesondere in Bezug auf die Höhe des Hilfsdruckes zu steuern, mit dem der Patient z. B. zur Vermeidung von OSA- oder UAR-Syndromen beaufschlagt wird. Hierbei ergibt sich der Vorteil, dass die an sich bekannte CPAP-Therapie schonend lediglich beim Auftreten gefährlicher Atmungszustände mit einem erhöhten Druck erfolgt. Es ergibt sich eine erhebliche Entlastung des Patienten.

Gemäß einer weiteren bevorzugten Ausführungsform der Erfindung wird der mindestens eine Atmungsparameter zur Steuerung eines Schlafüberwachungsgerätes verwendet, das zur Signalisierung und/oder Registrierung von Atmungsstörungen eingerichtet ist. Es wurde festgestellt, dass die erfindungsgemäß ermittelten Atmungsparameter geeignet sind, bei Vergleich mit Referenzwerten von gesunden Probanden zuverlässige Aussagen über zeitweilige Verengungen oder Verschließungen der oberen Atemwege eines schlafenden Probanden zu liefern. Gegenstand einer Weiterbildung der Erfindung ist somit eine Vorrichtung zur Signalisierung und/oder Registrierung von Schlafunterbrechungen durch Atmungsstörungen. Es sind vorzugsweise Signalisierungen mit visuellen Signalen (Leucht- oder Bildschirmanzeige, Protokolldrucker) oder auch Schallsignalen (Alarm) vorgesehen.

Erfindungsgemäße Vorrichtungen sind insbesondere Beatmungsgeräte mit steuerbaren Druckerzeugern und Schlafüberwachungsgeräte mit anwendungsabhängig gewählten Signalisierungseinrichtungen. Das Schlafüberwachungsgerät kann selbst Teil des Beatmungsgerätes sein. Der Druckerzeuger und/oder die Signalisierungseinrichtung werden in Abhängigkeit von den erfindungsgemäß erfassten Atmungsparametern und vorbestimmten Referenzwerten betätigt. Das Beatmungsgerät bildet einen Regelkreis, in dem laufend die jeweilige Zustandsgröße des Herz-Kreislauf-Systems gemessen, analysiert und mit den Referenzwerten verglichen wird. In Abhängigkeit vom Ergebnis des Vergleiches mit den Referenzwerten wird der Druckerzeuger eingestellt. Entsprechend enthält eine erfindungsgemäße Vorrichtung eine Messeinrichtung, einen Prozessorkreis und eine Stelleinrichtung für den Druckerzeuger.

Die Erfindung besitzt die folgenden Vorteile. Die erfindungsgemäß ermittelten Atmungsparameter eröffnen die Möglichkeit für eine zuverlässige und reproduzierbare Schlafüberwachung. Es ist eine einfache Signalverarbeitung vorgesehen. Die Belastung des Probanden durch die Messwertaufnahme ist gering. Erfindungsgemäße Beatmungs- und/oder Schlafüberwachungsgeräte können für den klinischen Bereich (Schlaflabor) oder auch für die persönliche Verwendung durch Einzelpersonen ausgelegt sein. Die Erfindung ist universell bei beliebigen Patienten, insbesondere unabhängig vom Alter des Patienten, umsetzbar. Erfindungsgemäß wird eine einfach zu handhabende Methode bereitgestellt, mit der das UAR-Symptom erkennbar ist. Bei Krankheitssymptomen, die denen von Patienten mit OSA gleichen, wäre ein UAR-Symptom mit herkömmlichen Mitteln nur durch aufwendige Ösophagusdruckmessungen erfassbar, was erfindungsgemäß vermieden wird.

Weitere Einzelheiten und Vorteile der Erfindung werden im folgenden unter Bezug auf die beigefügten Zeichnungen beschrieben. Es zeigen:

Fig. 1:     ein Flussdiagramm zur Illustration der erfindungsgemäßen Erfassung von Atmungsparametern,

Fig. 2:     ein Flussdiagramm zur Illustration von Einzelheiten der erfindungsgemäßen Rhythmusanalyse,

Fign. 3 bis 5:   Kurvendarstellungen mit Mess- und Auswertungsergebnissen und

Fig. 6:     eine schematische Übersichtsdarstellung einer Ausführungsform erfindungsgemäßer Beatmungs- und/oder Schlafüberwachungsgeräte.

Ausführungsformen erfindungsgemäßer Verfahren

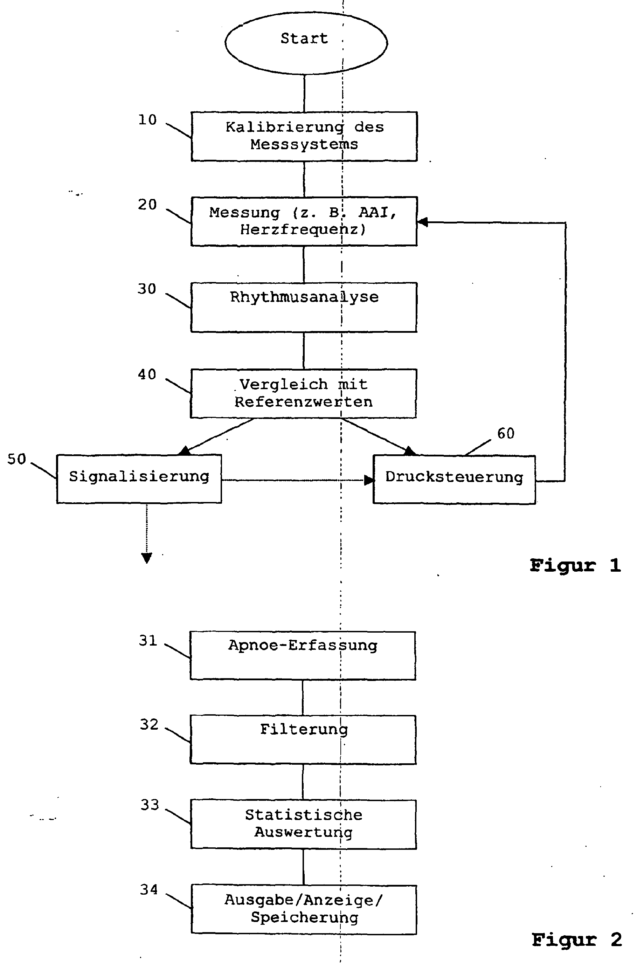

Eine Übersichtsdarstellung verschiedener Varianten der Umsetzung und Anwendung der erfindungsgemäßen Erfassung von Atmungsparametern ist in Fig. 1 gegeben. Die wesentlichen Schritte der Parametererfassung sind die Messung 20 mindestens einer Zustandsgröße und die Rhythmusanalyse 30. Ein Vergleich 40 der erfassten Atmungsparameter mit Referenzwerten mit einer anschließenden Signalisierung 50 und/oder Drucksteuerung 60 sind bevorzugte Anwendungen der Erfindung. Die zu Verfahrensbeginn angegebene Kalibrierung 10 ist ein fakultatives Merkmal der Erfindung, die anwendungsabhängig einmalig oder wiederholt probandenbezogen durchgeführt wird.

Die Messung 20 umfasst die messtechnische Aufnahme des zeitlichen Verlaufs mindestens einer Zustandsgröße des Herz-Kreislauf-Systems des Probanden, die sich mit der Atmung korreliert verändert. Es erfolgt beispielsweise die direkte oder indirekte Messung des Atemflusses oder der Herzaktivität. Der Atemfluß wird mit einem Atemflusssensor, z. B. indirekt mit einem Thermistor, gemessen. Die Herzaktivität wird durch eine hochauflösende Langzeitaufzeichnung eines Elektrokardiogramms (EKG) ermittelt. Es werden die R-R-Abstände im EKG registriert und aus diesen der zeitliche Verlauf der Atemzüge reproduziert. Aus dem gemessenen Zeitverlauf der jeweiligen Zustandsgröße werden die Atemzug-Atemzug-Intervalle oder -abstände (AAI) in Abhängigkeit von der Zeit ermittelt und gegebenenfalls die Stärke der Atemzüge (Atemfluß) abgeschätzt.

Die AAI-Messung aus den Atemflusssignalen erfolgt vorzugsweise durch Detektion der Minima der Atemflusssignale und Ermittlung von deren zeitlichen Abstand. Der Abstand zwischen zwei Minima wird jeweils als AAI-Wert verwendet. Alternativ kann der AAI-Wert auch aus dem zeitlichen Abstand der Nullwerte des Atemflusses oder der Maxima ermittelt werden. Die Minimadetektion wird jedoch wegen der erhöhten Zuverlässigkeit bevorzugt, die insbesondere für die anschließende statistische Auswertung (siehe Schritt 30) von Bedeutung ist.

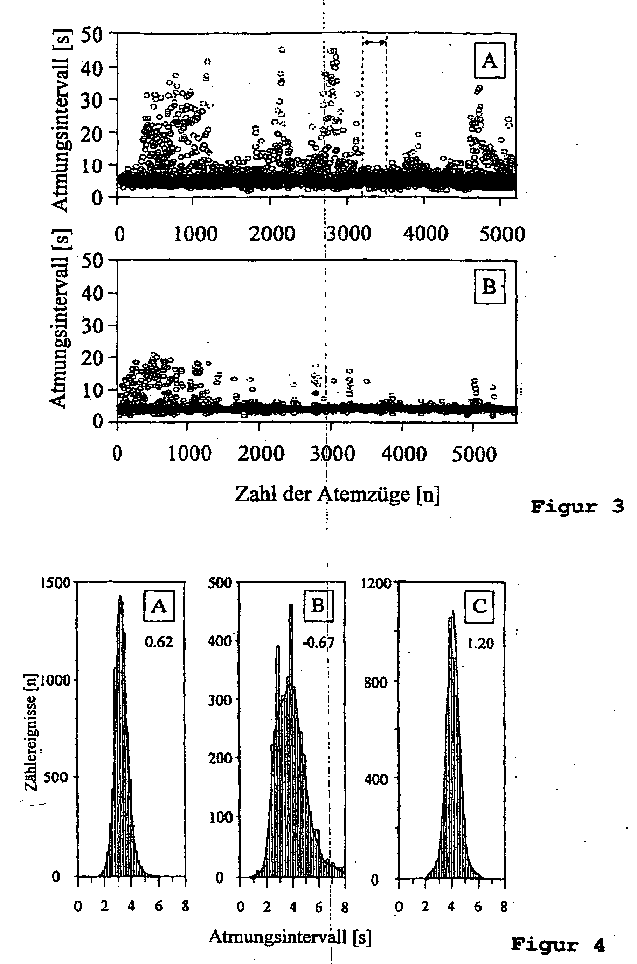

In Fig. 3 sind die gemessenen Atmungsintervalle eines erkrankten Probanden A und eines behandelten Probanden B (siehe unten) im Vergleich dargestellt. Die Atmungsintervalle (AAI) wurden für die Dauer von rund 8 Stunden aufgenommen. Bei einem typischen Atmungsintervall von rund 6 Sek. ergeben sich etwa 5.000 Atemzüge. Es zeigen sich, insbesondere bei dem kranken Probanden A, häufig Atmungsintervalle, die erheblich länger als 10 s betragen. Es zeigen sich Unterbrechungen der Atmung von bis zu 45 s. Bei der Rhythmusanalyse 30 erfolgt eine Verarbeitung des Zeitverlaufs der jeweils gemessenen Zustandgröße und eine statistische Auswertung. Einzelheiten dieser Schritte sind in Fig. 2 schematisch illustriert. Die in Fig. 2 gezeigte Prozedur zur Verarbeitung und Auswertung von Messgrößen ist ein Gegenstand der vorliegenden Erfindung.

Bei der Rhythmusanalyse 30 erfolgen zunächst ggf. eine Apnoe-Erfassung 31 (nicht zwingend vorgesehen), anschließend eine Filterung 32 der nicht obstruierten Atmung, anschließend eine statistische Auswertung 33 und schließlich eine Ausgabe, Anzeige und/oder Speicherung 34 der bei Schritt 33 ermittelten Atmungsparameter.

Bei der Apnoe-Erfassung 31 werden alle Atmungsintervalle ermittelt, die länger dauern als ein vorbestimmter Apnoe-Schwellwert. Dieser Schwellwert beträgt beispielsweise 10 s. Aus der zeitlichen Dichte der Apnoe-Zustände wird der Apnoe-Index AI ermittelt. Der Apnoe-Index ist gleich der Anzahl der erfassten Apnoe-Zustände pro Stunde. Des weiteren wird der Mittelwert und die Standardabweichung über die Dauer aller Apnoe-Zustände ermittelt.

Im Rahmen von Schritt 31 kann auch eine Hypopnoe-Erfassung mit einer Ermittlung des Apnoe-Hypopnoe-Index AHI vorgesehen sein. Ein Hypopnoe-Zustand ist gegeben, wenn der bei der Messung ermittelte oder aus der Messung geschätzte Atemfluss länger als ein vorbestimmter Hypopnoe-Schwellwert (z. B. 10 s) unterhalb von einen Grenzwert (z. B. 50 %) fällt und mit einer Mindestverringerung der Sauerstoffsättigung (z. B. 4 %) verbunden ist. Der AHI ergibt sich als Gesamtzahl der Apnoe- und Hypopnoe-Zustände pro Stunde.

Die Filterung 32 ist darauf gerichtet, alle Atemzüge zu ermitteln, bei denen eine nicht-obstruierte Atmung erfolgt. Alle Atmungsintervalle werden mit einem vorbestimmten Filterwert verglichen. Die Atmungsintervalle, die kürzer als der Filterwert sind, werden der normalen, nicht-obstruierten Atmung zugeordnet. Der Filterwert wird beispielsweise als Absolutwert (z. B. 10 s, siehe Schritt 31) vorgegeben. In Fig. 3 (oberer Teil) ist mit dem Doppelpfeil ein längerer Zeitbereich markiert, in dem eine nicht obstruierte Atmung erfolgt.

Bei Schritt 33 werden die gefilterten Atemzüge mit normaler, nicht obstruierter Atmung einer statistischen Auswertung unterzogen. Die statistische Auswertung setzt das Vorliegen einer ausreichenden Anzahl von Messwerten voraus. Vorteilhafterweise hat sich gezeigt, dass die Erfindung bereits nach kurzen Messzeiten im Minuten-Bereich, z. B. rund 10 min, zuverlässige Ergebnisse liefert. Zur statistischen Auswertung werden nicht nur zusammenhängende Gruppen von Atemzügen berücksichtigt, sondern gegebenenfalls auch die Atemzüge, die zu verschiedenen, durch Apnoe-Zuständen getrennten Gruppen von Atemzügen gehören. Im einzelnen werden der Mittelwert und die Standardabweichung der Atmungsintervalle ermittelt. Des weiteren wird vorzugsweise ein Normalbereich der Atmung definiert, in den alle Atmungsintervalle mit einer Dauer innerhalb von $\pm$ 50 % des Mittelwerts fallen. Dadurch wird vermieden, dass das Ergebnis der Auswertung durch vereinzelte starke Messschwankungen ("Ausreißer") verfälscht wird. Alle Atmungsintervalle, die in den Normalbereich fallen, werden dann als sogenannte Normalintervalle zur Berechnung der Standardabweichung und des Kurtosis-Wertes herangezogen. Die statistischen Größen können alternativ aber auch auf der Grundlage aller Atmungsintervalle mit nicht-obstruierter Atmung ermittelt werden. Der Kurtosis-Wert $\delta$ (oder sog. Exzess-Wert) ist wie folgt definiert:

$$\delta = [n \sum_{i=1}^{n}(x_i - \overline{x})^4 / (\sum_{i=1}^{n}(x_i - \overline{x})^2)^2] - 3$$

Dabei stellt $\overline{x}$ den Mittelwert aus den n (n: natürliche Zahl) betrachteten, in der Verteilung enthaltenen Messwerten $x_i$ dar.

Der Kurtosis-Wert einer Normalverteilung ist gleich Null. Ein positiver Kurtosis-Wert bedeutet eine im Vergleich zur Normalverteilung schärfere Verteilung, ein negativer Kurtosis-Wert hingegen eine verbreiterte Verteilung.

Die Erfinder haben festgestellt, dass insbesondere der Kurtosis-Wert und die Standardabweichung der Atmungsintervalle als erfindungsgemäß ermittelte Atmungsparameter mit dem AHI-Wert korrelieren und damit für den Schlafzustand des Probanden charakteristisch sind. Die statistische Auswertung 33 erfolgt unter Verwendung von an sich bekannten statistischen Verfahren und Programmwerkzeugen. Die ermittelten Atmungsparameter (Kurtosis-Wert und/oder Standardabweichung) werden bei Schritt 34 für die weitere Bearbeitung ausgegeben, angezeigt (z. B. visuell auf einem Bildschirm oder ausgedruckt) und/oder gespeichert. Es kann auch eine Umrechnung zur Anpassung an Folgeschritte bei der Verwendung der Atmungsparameter vorgesehen sein.

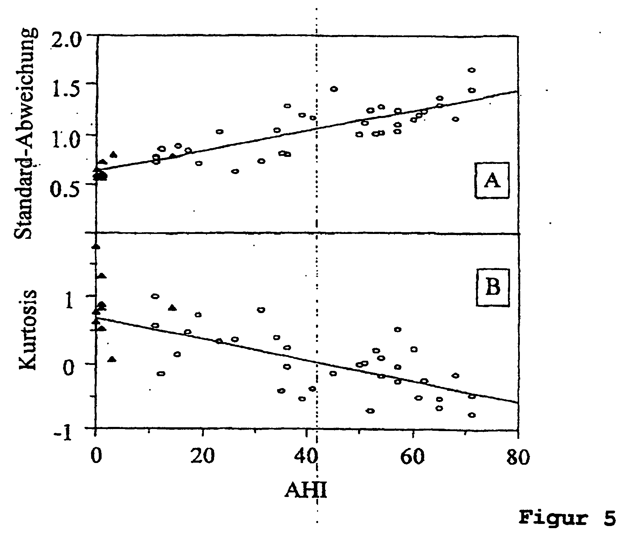

Die Fign. 4 und 5 illustrieren die statistische Auswertung 33 beispielhaft. Fig. 4 zeigt die Verteilung der berücksichtigten Atmungsintervalle für einen gesunden Probanden A, einen OSA-Patienten B und einen behandelten OSA-Patienten C. Das Atmungsmuster des gesunden Probanden A ist sehr gleichförmig. Es treten keine Apnoe-Zustände auf. Die Standardabweichung der Dauer sämtlicher (nicht-obstruierter) Atemzüge ist gering. Es zeigt sich eine enge Verteilung der Normalintervalle. Dementsprechend ist der Kurtosis-Wert größer als 0 (hier z. B.: 0.62). Demgegenüber ist das Atmungsverhalten des OSA-Patienten B sehr unkontinuierlich (siehe auch Fig. 3, obere Abbildung). Es zeigt sich, dass auch das nicht-obstruierte Atmen des kranken Probanden verändert ist. Die Standardabweichung der Normalintervalle ist erhöht. Außerdem ist die Häufigkeitsverteilung der Normalintervalle verändert, wie auch dem Vergleich der linken und mittleren Abbildungen in Fig. 4 zu entnehmen ist. Der Kurtosis-Wert ist erheblich verringert (z. B.: - 0.67) .

Wie in Fig. 5 dargestellt ist, besteht eine signifikante positive Korrelation zwischen den Standardabweichungen der nicht-obstruierten Atmungsintervalle und den AHI-Werten (oberer Teil von Fig. 5) und eine ausgeprägte negative Korrelation zwischen den AHI-Werten und Kurtosis-Werten der Atemzug-Häufigkeitsverteilungen (unterer Teil von Fig. 5). Es zeigt sich, dass mit zunehmendem AHI-Wert, d. h. insbesondere mit relativ zu den Hypopnoe-Zuständen zunehmender Anzahl von Apnoe-Zuständen, die Standardabweichungen zunehmen (größere Variabilität der Atmungsintervalle) und die Kurtosis-Werte abnehmen (zunehmende Abweichung der Häufigkeitsverteilung der Normalintervalle von einer Normalverteilung). Auf diesem überraschenden Befund basieren Anwendungen des erfindungsgemäßen Verfahrens bei der Beatmung und/oder Schlafüberwachung, die im folgenden unter Bezug auf die Schritte 40, 50 und 60 in Fig. 1 erläutert werden.

Die genannten Korrelationen zwischen den erfindungsgemäß ermittelten Atmungsparametern und Schlafzuständen, die sich durch einen erhöhten AI- oder AHI-Wert auszeichnen, ermöglichen allein auf der Grundlage eines Vergleiches (Schritt 40 in Fig. 1) mit Referenzwerten eine Signalisierung des Schlafzustandes und/oder eine Einstellung eines Beatmungsgerätes. Ein Verfahren zur Analyse von Schlafzuständen mit der genannten Erfassung von mindestens einem Atmungsparameter und einem Vergleich mit mindestens einem Referenzwert ist auch Gegenstand der Erfindung. Beim Vergleich 40 wird der aktuell ermittelte Wert der Atmungsparameter mit einem vorbestimmten, gespeicherten Referenzwert verglichen. Falls z. B. die Standardabweichung den Referenzwert überschreitet oder der Kurtosis-Wert den Referenzwert unterschreitet, wird ein Stellsignal erzeugt, mit dem die Schritte Signalisierung 50 und/oder Drucksteuerung 60 gestartet werden. Als Referenzwert werden vorab gespeicherte, universelle Referenzwerte (Absolutwerte von gemessenen gesunden Probanden) verwendet.

Wird bei Schritt 40 das Stellsignal erzeugt, so wird mit Schritt 50 ein Schallsignal, eine Anzeige oder ein gedrucktes Protokoll erzeugt. Diese Signalisierung richtet sich an einen Operator des jeweiligen Steuergerätes z. B. im Schlaflabor oder an den Probanden selbst.

Das erfindungsgemäße Analyseverfahren mit einer Signalisierung von Schlafzuständen mit obstruierter Atmung kann durch eine erfindungsgemäße Vorrichtung (siehe unten) und/oder ein Computerprogramm umgesetzt werden.

Die vorteilhafte Wirkung der Steuerung des Druckerzeugers eines Beatmungsgerätes in Abhängigkeit von den ermittelten Atmungsparametern ist in den Fign. 3 (untere Abbildung) und 4 (rechte Abbildung) gezeigt. Ein OSA-Patient wird über eine nasale Maske mit einem Beatmungsgerät (CPAP-Gerät) verbunden. Die Einstellung oder Erhöhung des dem Patienten zugeführten Überdrucks in Abhängigkeit vom Ergebnis des Vergleiches 40 bewirkt eine erhebliche Verringerung der Zahl von Atemaussetzern und deren deutliche Verkürzung (siehe Fig. 3) und eine Verringerung der Variabilität des nicht-obstruierten Atmens und eine deutliche Erhöhung des Kurtosis-Wertes (siehe behandelter Patient C in Fig. 4).

Ausführungsformen erfindungsgemäßer Vorrichtungen

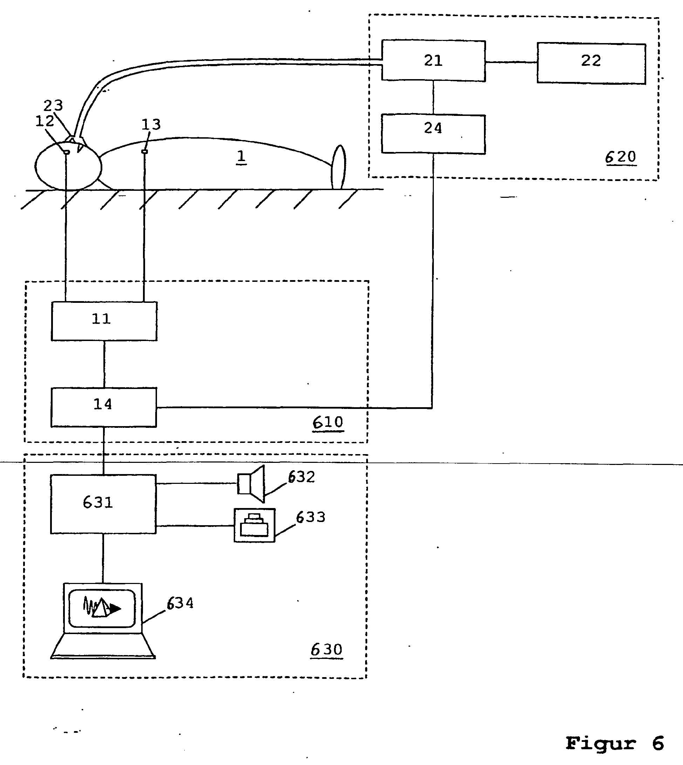

In Fig. 6 sind Komponenten eines erfindungsgemäßen Beatmungs- und Schlafüberwachungsgerätes schematisch dargestellt. Gemäß abgewandelten Ausführungsformen der Erfindung kann auch der ausschließliche Aufbau eines Überwachungs- oder Beatmungsgerätes vorgesehen sein. Im einzelnen zeigt Fig. 6 eine Steuereinrichtung 610, eine Beatmungseinrichtung 620 und eine Signalisierungseinrichtung 630.

Die Steuereinrichtung 610 umfaßt eine Messeinrichtung 11, die mit Sensoren 12, 13 am Patienten verbunden ist, einen Prozessorkreis 14 und eine Stelleinrichtung 15. Als Sensoren kann allgemein jeder Messaufnehmer verwendet werden, mit dem die Atmungsintervalle messbar sind (z. B. nasale Druckaufnehmer, Pneumotachographen, CPAP-Fluß/Druck-Sensoren, Thermoelemente, akustische Sensoren o. dgl.). Die Sensoren 12, 13 sind beispielsweise ein Thermistor und eine EKG-Elektrodenanordnung. Die Messeinrichtung ist zur Umsetzung der oben genannten Schritte 10 und 20 vorgesehen und enthält ein an sich bekanntes Atemfluss-Messgerät und/oder ein EKG-Gerät. Die Messergebnisse werden an den Prozessorkreis 14 gegeben, der zur Umsetzung der oben genannten Schritte der Rhythmusanalyse 30 eingerichtet ist. Der Prozessorkreis 14 ist beispielsweise ein Mikroprozessor eines Computers, in dem die Rhythmusanalyse 30 mit dem genannten Computerprogramm durchgeführt wird. Es ist alternativ aber auch eine schaltungsmäßige Umsetzung der Rhythmusanalyse 30 mit Komparator- und Filterschaltungen zur Umsetzung der oben genannten Schritte 31 und 32 und Rechenschaltungen für die statistische Auswertung 33 vorgesehen. Des weiteren kann der Prozessorkreis mit einem Speicher, Anzeigegeräten und/oder Ausgabegeräten verbunden sein. Anwendungsabhängig kann der Prozessorkreis 14 auch zur Umsetzung des Vergleichsschrittes 40 und zur Bereitstellung des Stellsignales bei der Feststellung obstruierter Atmung eingerichtet sein. Das Stellsignal wird an die Stelleinrichtung 24 gegeben, mit der die Beatmungsvorrichtung 620 betätigt wird.

Die Beatmungseinrichtung 620 umfaßt ein an sich bekanntes CPAP-Gerät 21 mit einem Druckerzeuger, gegebenenfalls eine zusätzliche Steuerschaltung 22 und die Stelleinrichtung 24. Das CPAP-Gerät 21 ist über eine Schlauchverbindung und eine Nasal- und/oder Oralmaske 23 mit dem Patienten 1 verbunden. Bei Erzeugung des Stellsignals durch den Prozessorkreis 14 wird der Druck des CPAP-Gerätes 21 erhöht. Die Erhöhung erfolgt zunächst mit einem vorbestimmten Druckintervall (z. B. 1 mbar), das gegebenenfalls im weiteren Messverlauf weiter erhöht wird. Es kann umgekehrt auch eine Absenkung des Druckes vorgesehen sein. Bei der CPAP-Steuerung kann eine kurzfristige (z. B. in Zeitintervallen von rd. 10 min je nach den ermittelten Atmungsparametern) oder längerfristige Druckänderung (z. B. Optimierung von Nacht zu Nacht) vorgesehen sein.

Die Signalisierungseinrichtung 630 enthält ebenfalls eine Stelleinrichtung 631, mit der in Abhängigkeit vom Stellsignal vom Prozessorkreis 14 ein Schallgeber 632, ein Drucker 633 und/oder eine Anzeige 634 betätigt werden.

Die Signalisierungseinrichtung 630 wird vorzugsweise zur Abgabe eines optischen Signals oder auch eines Schallsignals verwendet, wenn in Abhängigkeit von den erfindungsgemäß erfassten Atmungsparametern die Schlafüberwachung ein UAR-Symptom ergibt.

Die in der vorstehenden Beschreibung, den Zeichnungen und den Ansprüchen offenbarten Merkmale der Erfindung können sowohl einzeln als auch in beliebiger Kombination für die Verwirklichung der Erfindung in ihren verschiedenen Ausgestaltungen von Bedeutung sein.

**Patentansprüche**

1. Vorrichtung zur Beatmung eines Patienten und/oder zur Signalisierung von Schlafzuständen, mit:

- einer Steuereinrichtung (610),
die zur Erfassung von mindestens einem Atmungsparameter aus jeweils die zeitliche Dauer eines Atemzugs darstellenden gemessenen Atmungsintervallen eingerichtet ist, der für den Atmungszustand eines schlafenden Probanden charakteristisch ist und durch die Variabilität der Atmungsintervalle in Phasen nicht-obstruierter Atmung und/oder davon abgeleitete statistische Größen gebildet wird,
und die einen Vergleicher zum Vergleich des mindestens einen Atmungsparameters mit einem Referenzwert enthält,
wobei die Steuereinrichtung (610) zur Erzeugung eines Stellsignals in Abhängigkeit vom Ergebnis des Vergleiches eingerichtet ist, und
- einer Beatmungseinrichtung (620) und/oder einer Signalisierungseinrichtung (630), die mit dem Stellsignal steuerbar sind.

2. Vorrichtung gemäß Anspruch 1, bei der die Beatmungseinrichtung (20) einen mit dem Stellsignal steuerbaren Druckerzeuger enthält.

3. Vorrichtung gemäß Anspruch 1 oder 2, bei der die Beatmungseinrichtung (20) ein CPAP-Gerät (21) enthält.

4. Vorrichtung gemäß einem der Ansprüche 1 bis 3, bei der eine Messeinrichtung (11) vorgesehen ist, die mit Sensoren (12, 13) am Probanden verbunden und zur Messung des zeitlichen Verlaufs mindestens einer sich wiederholt mit der Atmung verändernden Zustandsgröße des Herz-Kreislauf-Systems des Probanden eingerichtet ist.

**5.** Vorrichtung gemäß Anspruch 4, bei der die Sensoren einen Thermistor (12) und/oder eine EKG-Elektrodenanordnung (13) umfassen.

**6.** Vorrichtung gemäß einem der Ansprüche 1 bis 5, bei der die Signalisierungseinrichtung (30) einen Schallgeber (32), einen Drucker (33) und/oder eine Anzeige (34) aufweist.

**Claims**

**1.** A device for artificially ventilating a patient and/or for signaling sleep stages, comprising:

- a control device (610)
which is designed to detect at least one breathing parameter from measured breath-to-breath intervals each representing the duration of one breath, said parameter being characteristic for the breathing status of a sleeping individual and being provided by the variability of the breath-to-breath intervals in phases of unobstructed breathing and/or statistical variables derived therefrom,
and which comprises a comparator for comparing said at least one breathing parameter to a reference value, the control device (610) being designed to generate an actuating signal as a function of the result of the comparison, and
- a breathing apparatus (620) and/or a signaling device (630) which can be controlled by the actuating signal.

**2.** The device as in Claim 1, in which the breathing device (20) comprises a pressur generator which can be controlled by means of the actuating signal.

**3.** The device as in Claim 1 or 2, in which the breathing device (20) comprises a CPAP device (21).

**4.** The device as in one of Claims 1 through 3, comprising a measuring device (11) which is connected to sensors (12,13) on the individual and which is designed so as to allow the measurement of the derivative trend with respect to time of at least one variable of state of the cardiovascular system of the individual, which variable recurrently changes with the respiration.

**5.** The device as in Claim 4, in which the sensors comprise a thermistor (12) and/or an ECG electrode arrangement (13).

**6.** The device as in any one of Claims 1 through 5, in which the signaling device (30) comprises a sound generator (32), a printer (33) and/or a display (34).

**Revendications**

**1.** Dispositif de ventilation artificielle d'un patient et/ou de signalisation d'états du sommeil, comportant :

- un dispositif de commande (610),
lequel est destiné à déterminer au moins un paramètre de respiration à partir de chacun des intervalles de temps mesuré, représentant la durée d'un souffle, lequel paramètre est caractéristique de l'état de respiration d'un patient endormi et est formé par la variabilité des intervalles de respiration en phases de respiration non obstruées et/ou par des grandeurs statistiques qui en découlent,
et lequel comporte un comparateur destiné à comparer ledit au moins un paramètre de respiration avec une valeur de référence, ledit dispositif de commande (610) étant conçu pour générer un signal de commande en fonction du résultat de la comparaison, et
- une unité de ventilation artificielle (620) et/ou une unité de signalisation (630), qui peuvent être commandées par le signal de commande.

**2.** Dispositif selon la revendication 1, dans lequel l'unité de ventilation artificielle (620) comporte un générateur de pression apte à être commandé par le signal de commande.

**3.** Dispositif selon la revendication 1 ou 2, dans lequel l'unité de ventilation artificielle (620) comprend un appareil CPAP (21).

**4.** Dispositif selon l'une quelconque des revendications 1 à 3, dans lequel il est prévu un dispositif de mesure (11), qui est relié à des capteurs (12, 13) au niveau du patient et est conçu pour mesurer l'évolution dans le temps d'au moins une grandeur d'état, variant de manière répétée avec la respiration, du système cardio-vasculaire du patient.

**5.** Dispositif selon la revendication 4, dans lequel les capteurs sont formés par un thermistor (12) et/ou un système d'électrodes d'électrocardiogramme (13).

**6.** Dispositif selon l'une quelconque des revendications 1 à 5, dans lequel l'unité de signalisation (630) comporte un transmetteur de sons (32), une imprimante (33) et/ou un écran d'affichage (34).

Figur 1

Figur 2

Figur 3

Atmungsintervall [s]

Figur 4

Figur 5

21

22

23
12

13

1

24

*620*

11

14

*610*

632

631

633

*630*

634

**Figur 6**